# EUROPEAN PATENT APPLICATION

(11) **EP 3 130 303 A1**
(43) Date of publication of application: **15.02.2017**
(21) Application number: 15776233.7
(22) Date of filing: 27.03.2015
(51) Int. Cl.: A61B 18/12

(54) **PLASMA TREATMENT SYSTEM**

(30) Priority: 11.04.2014 JP 2014082286
(71) Applicant: Olympus Corporation, Hachioji-shi, Tokyo 192-8507 (JP)
(72) Inventor: ISHIKAWA, Manabu, Hachioji-shi, Tokyo 192-8507 (JP); KIMURA, Shuichi, Hachioji-shi, Tokyo 192-8507 (JP); WATANABE, Koichiro, Hachioji-shi, Tokyo 192-8507 (JP)
(74) Representative: Gunzelmann, Rainer
(86) International application number: PCT/JP2015/059754
(87) International publication number: WO 2015/156156

(57) **Abstract**

In a plasma treatment system, a coefficient K which is a ratio of a supply amount and a suction amount of saline and also a minimal water amount required for generation of plasma is provided, and the required supply amount and suction amount of the saline are set from the coefficient K acquired on the basis of an output level of high-frequency energy, thereby performing a plasma treatment.

## Description

### BACKGROUND OF THE INVENTION

### 1. Field of the Invention

The present invention relates to a plasma treatment system which is used together with a plasma treatment tool.

### 2. Description of the Related Art

Generally, in a semiconductor manufacturing technology and others, there has been widely known a technology which gives an etching treatment to a desired region by discharge of plasma or the like generated between electrodes in a vacuum atmosphere. In a medical technology, various medical devices belonging to a high-frequency treatment tool using this discharge technology have been recently realized. For example, in Non-patent Literature 1 (Plasma Characteristics of Repetitively-Pulsed Electrical Discharges in Saline Solutions Used for Surgical Procedures, Jean Woloszko et.al: IEEE TRANSACTIONS ON PLASMA SCIENCE, VOL. 30, JUNE 2002), a technology of a probe concerning a treatment tool using plasma (which will be referred to as a plasma treatment tool hereinafter) in which an active electrode and a return electrode are arranged on a probe tip is disclosed. This treatment tool is a bipolar treatment tool from a viewpoint of an electrode configuration, and can reduce a current flowing through a body tissue as compared with a monopolar type. Specifically, there are provided cylindrical active (titanium) electrodes arranged on a central side on a tip surface of the probe and return (stainless steel) electrodes arranged to surround the periphery of the former electrodes.

This treatment is called low-temperature ablation, and included in a low-heat invasive treatment. Specifically, this is a treatment by which saline in which plasma is generated is brought into contact with a body tissue and a surface of the body tissue is subjected to transpiration by energy of this plasma. This low-temperature ablation treatment is preferable for, e.g., a tonsillectomy, and is said to have an effect of reducing pain or alleviating swelling or complications after a surgical operation to a patient if a treatment equivalent to a conventional cautery knife is performed.

As a shape of the probe, in case of a bipolar type, an electrode pair is provided on a tip side, but various kinds of shapes, e.g. , those for excising or incising a surface of a body tissue have been realized in correspondence with a use application of a treatment to the body tissue.

Further, Patent Literature Jpn. Pat. Appln. KOKAI Publication No. 2010-42249 proposes a surgical instrument to join held body tissues. As to this surgical instrument, there is disclosed a mechanism which supplies saline to a grip section at a tip in a state where a body tissue is held.

There is proposed a technology which performs discharge by immersing a tip surface of this probe into a conductive liquid solution having high electric conductivity, e.g., saline and suppling an alternating voltage (current) power having an arbitrary high frequency to an active electrode, thereby generating plasma.

The above-described plasma treatment tool uses the conductive liquid solution, e.g., saline having the high electric conductivity (electric conductive properties) at the time of generating the plasma. When a treatment to transpire a body tissue is performed by using this plasma, an unnecessary transpired body tissue piece is mixed in the saline as an impurity of a very small size. When the impurity is mixed in the saline in this manner, characteristic such as an impedance vary, and hence a situation like extinction of the plasma may occur depending on conditions.

To stably continue a plasma generating state, it is preferable to constantly maintain the saline in an environment close to that at the time of generating the plasma, the pure saline must be freshly supplied, and the saline containing impurities must be eliminated (sucked).

As a publicly known plasma treatment tool, a configuration in which both a supply port and a suction port are provided on a probe tip side and a tube is inserted into the treatment tool so that the treatment tool can be coupled with an external pump apparatus has been realized. The probe of this plasma treatment tool has a configuration in which the supply portion from which saline is supplied and the suction port from which the saline is sucked and removed are provided in a tip surface where electrodes (an active electrode and a return electrode) are arranged or near this tip surface. Supply of the fresh saline and suction of the supplied saline are continuously performed to a plasma generating region.

In general, as regards of supply of the saline, a valve provided at a supply portion of a saline bag suspended from a support arm section is manually adjusted, and a supply amount which seems a moderate amount is supplied under its own weight.

As to the manual valve adjustment, an actual water amount dripping from the probe during a treatment is not measured, but the adjustment is performed while carrying out visual confirmation or watching an image displayed in a display screen, and hence an actual supply amount and suction amount are not grasped. That is, it is not easy to control a supply amount of the saline to a minimal required supply amount by the manual valve operation based on a rough estimate.

Furthermore, although a flow volume control section which controls a supply amount of the saline is present, to generate plasma to perform an appropriate treatment, an apparatus which carries out control while taking a supply amount and a suction amount of the saline into consideration has not been realized yet.

Thus, the present invention provides a plasma treatment system which can supply and suck a minimal required conductive liquid solution to and from a treatment tool which generates plasma near electrodes by allowing a high-frequency current to flow through the conductive liquid solution supplied between the two electrodes and performs a treatment to a body tissue.

### BRIEF SUMMARY OF THE INVENTION

According to an embodiment of the present invention, there is provided a plasma treatment system characterized by comprising: a treatment section which comprises a first electrode and a second electrode which forms a pair with the first electrode, and generates plasma in a conductive liquid solution, in which the first electrode and the second electrode are immersed, by using high-frequency energy supplied between the first electrode and the second electrode to give a treatment to a body tissue; a setting section which sets a predetermined ratio of a supply amount from a supplying section which supplies the conductive liquid solution for immersion between the first electrode, the second electrode, and a treatment target surface layer of the body tissue, and a suction amount from a sucking section which sucks the conductive liquid solution; a detecting section which detects a supply amount of the conductive liquid solution supplied from the supplying section, and a suction amount of the conductive liquid solution sucked into the sucking section; and a control section which controls the supply amount and the suction amount to the ratio set by the setting section on the basis of the supply amount and the suction amount detected by the detecting section.

Further, according to an embodiment of the present invention, there is provided a plasma treatment system which is a control system used together with a supplying/sucking section characterized by including: a plasma treatment tool having a probe which comprises at a tip portion thereof a first electrode and a second electrode to generate plasma from high-frequency energy; a high-frequency power supply which supplies the high-frequency energy to the first electrode and the second electrode; a supplying section which performs irrigation with a predetermined supply amount of a conductive liquid solution provided by a supply amount sensor so that both the first electrode and the second electrode are immersed in the conductive liquid solution; and a sucking section which sucks and discharges a predetermined suction amount of the conductive liquid solution that has been used to irrigate and has passed the first electrode and the second electrode from the suction port and that is provided by a suction amount sensor,
the system comprising a control section which compares a supply amount detected by the supply amount sensor with a suction amount detected by the suction amount sensor, performs feedback control, and controls the supply amount and the suction amount to reach a predetermined target amount within an immersion range to immerse both the first electrode and the second electrode.

Advantages of the invention will be set forth in the description which follows, and in part will be obvious from the description, or may be learned by practice of the invention. The advantages of the invention may be realized and obtained by means of the instrumentalities and combinations particularly pointed out hereinafter.

### BRIEF DESCRIPTION OF THE SEVERAL VIEWS OF THE DRAWINGS

The accompanying drawings, which are incorporated in and constitute a part of the specification, illustrate embodiments of the invention, and together with the general description given above and the detailed description of the embodiments given below, serve to explain the principles of the invention.
FIG. 1 is a block diagram showing an entire configuration of a plasma treatment system including a supply/suction unit;
FIG. 2 is a view showing a partial arrangement configuration of a tip portion of a probe in the plasma treatment apparatus;
FIG. 3 is a view showing a configuration of the supply/suction unit according to a first embodiment;
FIG. 4 is a view showing an amount of saline which is supplied and sucked to or from the probe during a treatment;
FIG. 5 is a flowchart for explaining a treatment method in the plasma treatment system;
FIG. 6 is a view showing an example of a ratio of a supply amount and a suction amount of saline provided by a plasma treatment tool system according to a second embodiment;
FIG. 7 is a view showing a structural example of a plasma treatment system according to a third embodiment;
FIG. 8 is a view showing a structural example of a plasma treatment system including a pH sensor according to a fourth embodiment;
FIG. 9A is a view showing an appearance configuration of a tip portion of a probe of a plasma treatment tool according to the fourth embodiment; and
FIG. 9B is a view showing a cross-sectional configuration of the probe shown in FIG. 9A.

### DETAILED DESCRIPTION OF THE PREFERRED EMBODIMENT

Embodiments according to the present invention will now be described hereinafter in detail with reference to the drawings.

### [First Embodiment]

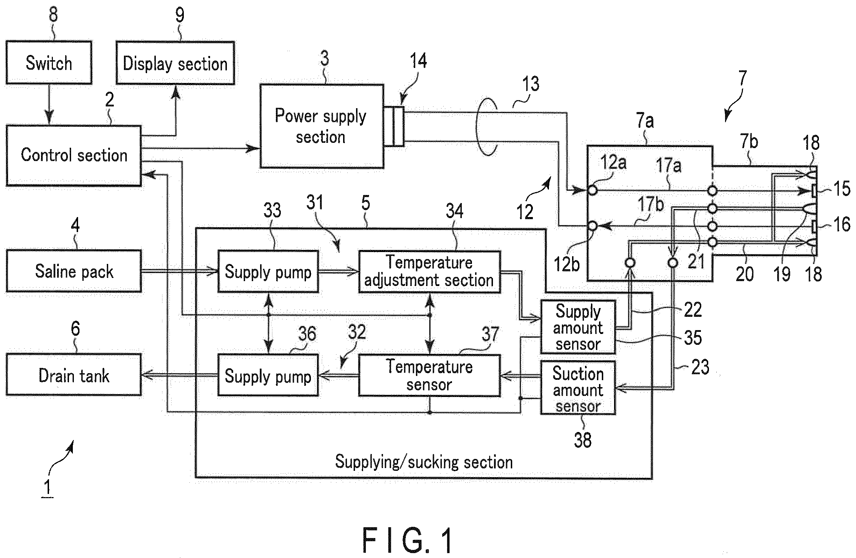

A first embodiment will now be described. FIG. 1 shows a block configuration of an entire plasma treatment system including a supplying/sucking section. This plasma treatment system 1 is used for a treatment of generating plasma and transpiring a body tissue as a treatment target by using this plasma. Since the body tissue is excised by using the plasma, thermal damage tc a periphery of an excised tissue is relatively small, and a low-invasive treatment is realized. The plasma treatment system 1 is used for excision of a tonsillar tissue or the like in, e.g. the field of otorhinolaryngology. Additionally, this plasma treatment system 1 may be used for excision of a synovial membrane, excision of a cartilage, and others in, e.g., the field of orthopedics, or may be used for excision of an organ (particularly, a liver) in abdominal operations in general.

The plasma treatment system 1 is roughly constituted of a control section 2 which controls the whole, a power supply section 3 which outputs high-frequency (RF) power, a saline bag 4 filled with saline which is a conductive liquid solution (or a conductive fluid), a supplying/sucking section 5 which performs later-described supply and suction of the saline, a drain tank 6 which accommodates the sucked saline, and a plasma treatment tool 7 which gives a treatment using plasma to a body tissue.

Although not shown, the control section 2 which controls the entire system includes a processing section having an arithmetic processing function and a memory storing a processing program, preset or appropriately set numeral figure information, a control table, and others, and has a function like that of a personal computer. Further, the control section 2 also includes an input section formed of an input panel, a keyboard or the like to input information or configure settings, and a display section 9 which displays information concerning treatments, set or detected information, and others. Furthermore, to the control section 2 are connected switches 8 to conduct a later-described plasma treatment, e.g. , a water feed switch, a drain switch, a footswitch, and others. The switches are switches to apply the high-frequency power on different output levels, and selected and operated by an operator in correspondence with treatment contents.

Moreover, a memory which stores an ID (an individual identification number) may be provided to the plasma treatment tool so that this ID is read out to the control section 2 when a cable is connected to the control section 2. In this case, a table storing setting conditions of plasma treatment tools having different specifications associated with IDs (e.g., a power supply voltage level to be supplied, a supply amount and a suction amount of the saline, and others) is set in the control section 2 in advance. With this configuration, an ID is designated at the time of connecting the cable, and setting conditions of a plasma treatment are selected from the table and set to an apparatus system (the control section 2 and the power supply section 3) without an input operation of the operator.

The power supply section 3 outputs high-frequency energy (which will be referred to as high-frequency power hereinafter) consisting of a high-frequency current (voltage) based on sine waves, pulse waves, or the like. The power supply section 3 has a turning function to reflected waves generated by a load (an impedance) of a high-frequency power application target. Since the power supply section 3 has this turning function, it is configured to select high-frequency power on output level, and switch them by control of the control section 2. For example, when driving power required in accordance with each treatment target or each type of a plasma treatment differs, an output level suitable for the treatment to be carried out is selected and set. Further, driving power to be supplied can be set in accordance with a type of a plasma treatment tool (e.g., a difference in shape of the active electrode). Furthermore, for example, a different output level can be set in accordance with a type of a treatment in e.g. , a transpiration and coagulation mode, a coagulation mode, or the like.

Moreover, when the memory storing the ID information is mounted in the plasma treatment tool and coupled with the power supply section 3, the ID information may be read from the treatment tool on the control section 2 side, and an output level may be automatically set without a setting operation of the operator using the input panel. As the saline bag 4, a commercially available one can be generally used.

The plasma treatment tool 7 will now be described.

The plasma treatment tool 7 is basically a treatment tool which is disposed of after use (a disposable type) and, as shown in FIG. 1, a grip section 7a and a probe 7b constitute a treatment tool main body (7) and form a pen-like shape. Although the plasma treatment tool 7 according to this embodiment is turned on and off at the time of a treatment by turning on and off the footswitch, an operation switch (a hand switch) (not shown) may be provided to the grip section 7a to turn on and off the power supply of the plasma treatment tool 7 at the time of a treatment.

Further, terminals 12a and 12b to take in the high-frequency power which is the high-frequency energy from the power supply section 3 are arranged at a rear end of the grip section 7a, and power supply cables 13 are detachably connected to these terminals 12. Furthermore, the power supply cables 13 are detachably connected to the power supply section 3 by using a connector 14. It is to be noted that, to prevent a high frequency from leaking to outside and prevent noise from overlapping the cables from the outside, use of electromagnetic shield cables or the like is preferable as the power supply cables 13.

Although this embedment is an example of integrally disposing of the used plasma treatment tool 7, the removed probe 7b alone can be disposed of if the grip section 7a and the probe 7b can be separately detached. Moreover, if the treatment tool main body and the power supply cables 13 can be separated from each other, the power supply cables 13 can be reused.

Additionally, as shown in FIG. 1, in the example of this embodiment, the probe 7b of the plasma treatment tool 7 has a cylindrical shape, and at least one active electrode (a first electrode) 15 and a return electrode (a second electrode) 16 are aligned within a tip surface 7c of the probe 7b. These electrodes 15 and 16 are electrically connected to the terminals 12a and 12b through inner wiring lines 17a and 17b. Here, a structural example of a so-called bipolar electrode is shown. As regards the bipolar electrode in this plasma treatment, a pair of electrodes are not arranged inside and outside a body tissue which is a treatment target like a monopolar type, but the respective electrodes are arranged to face one surface of the body tissue in a body cavity.

As a configuration of the bipolar electrode according to this embodiment, the active electrode and the return electrode are arranged to face each other at an extremely close range or arranged at positions close to each other on the tip surface of the treatment tool. These electrodes are configured to be electrically separated from each other by interposing a member made of an insulating material between the electrodes. Thus, since the generated plasma come into contact with a surface of the body tissue alone, it functions to reduce pain or alleviate swelling or complications after an operation to a patient.

As will be describe later, when the high-frequency power is supplied in a state where the active electrode 15 and the return electrode 16 are immersed in the saline used for irrigation, i. e. , when the switches 8 are ON, the high-frequency power is applied to the active electrode 15, and the plasma is generated between the active electrode 15 and the return electrode 16. In more detail, when a high-frequency current is allowed to flow through the saline present between the two electrodes 15 and 16, the saline is evaporated to create a vapor layer near the electrode 15. The plasma is considered to be produced by adding an electric field to this vapor layer.

It is to be noted that, when the plasma is moved closer to the body tissue, the body tissue can be transpired by a function of an OH radical or the like in the plasma.

Further, at the time of providing the respective electrodes, it is important to form these electrodes so that the return electrode 16 has a larger electrode surface area immersed in the saline than that of the active electrode 15. That is because, when the area of the return electrode 16 is smaller than that of the active electrode 15, the return electrode 16 plays the role of the active electrode 15, and the desired excellent plasma may not be produced.

Furthermore, supply ports 18 which are continuously arranged and have an arc-like shape are formed in an edge portion at a circumference of the tip surface 7c of the probe 7b, and a suction port 19 is opened below the active electrode 15. It is to be noted that the plasma treatment tool 7 has an electrical insulating coating applied thereto except the tip surface 7c of the probe 7b so that the high-frequency power does not leak to the outside. As wiring lines connected to the active electrode and the return electrode in the plasma treatment tool 7, electrically insulated high-frequency shield cables or the like having a shielded high frequency are used.

Moreover, as arrangement positions of the supply ports 18 and the suction port 19, positions sandwiching the active electrodes 15 and the return electrodes 16 therebetween are preferred. One end of each of inner tubes 20 and 21 is connected to each of the supply ports 18 and the suction port 19, and runs through the probe 7b. The inner tubes 20 and 21 are separably connected to outer tubes 22 and 23, respectively. In this embodiment, although the example where the inner tubes 20 and 21 are connected to the outer tubes 22 and 23 is shown, the inner tubes 20 and 21 and the outer tubes 22 and 23 may be one tube. When the inner tubes 20 and 21 and the outer tubes 22 and 23 are formed as separate members, the main body of the probe treatment tool 7 alone can be disposed of. It is to be noted that the return electrodes 16 and the supply ports 18 do not necessary have to be arranged on the tip surface 7c, and they may be arranged on a cylindrical side surface of the probe 7b. At least the supply ports 18 do not have to be restricted in particular as long as they are arranged on an outer peripheral side of the return electrodes 16 or the grip section 7a side and the active electrodes 15 and the return electrodes 16 can be immersed in the saline.

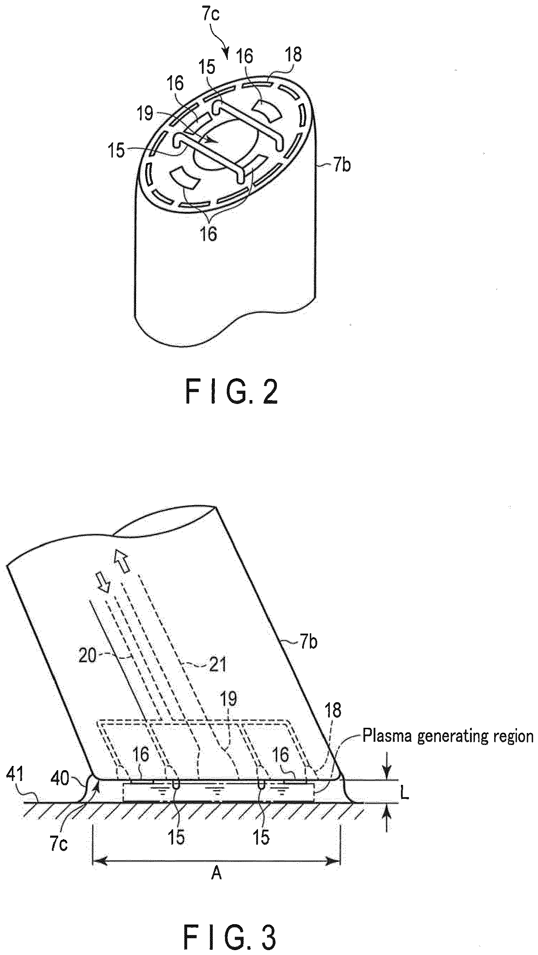

It is to be noted that the end surface 7c of the probe 7b shown in FIG. 1 is conceptually represented by a plane vertical to the outer peripheral surface. However, as shown in FIG. 2 and FIG. 3, the tip surface 7c is actually formed into a plane obliquely cut to the outer peripheral surface while considering operability so that an operator can grip the grip section to easily give a treatment. That is, the tip surface 7c of the probe 7b is formed to face a surface of the body tissue which is a treatment target in a parallel manner when the operator holds the plasma treatment tool 7 like a pen.

As shown in FIG. 2 and FIG. 3, saline 40 supplied through the tube 20 flows from the supply ports 18 for irrigation, and an irrigation layer is formed between the tip surface 7c of the probe 7b and a body tissue 41. Moreover, the saline is sucked from the suction port 19, and the saline is drained through the suction tube 21. At this time, the saline 40 which has flowed out from the supply ports 18 passes through the active electrodes 15 and the return electrodes 16 and is sucked into the suction port 19 together with the transpired body tissue.

In a state where the active electrodes (first electrodes) 15 and the return electrodes (second electrodes) 16 are immersed in the irrigation layer of the saline 40, when the high-frequency power is applied to the active electrodes 15, discharge occurs, and a plasma generating region is created. In one principle presumed at the present, the saline 40 is separated according to a molecular size in the plasma generating region, cations having high energy are generated, and the cations collide with the body tissue (e.g., an etching phenomenon in atmospheric pressure plasma) to destroy and chip away a tissue surface so that the tissue surface is separated from the body tissue 41 according to the molecular size. At the same time, the chipped-off body tissue is coagulated. The chipped-off and separated body tissue floats in the saline, and it is sucked into the suction port 19 with the saline 40 and then discharged. In addition, as plasma generating conditions, it is assumed that an application voltage/current of the high-frequency power varies depending on, e.g., a structural specification such as shapes or an inter-electrode distance of the active electrodes 15 and the return electrodes 16, characteristics of the conductive liquid solution (e.g., the saline) to be used, and others, and a unique specification is discovered at the time of design.

The supplying/sucking section 5 will now be described in detail.

The supplying/sucking section 5 shown in FIG. 1 performs supply and drainage (suction) of the saline (the conducive liquid solution) to and from the plasma treatment tool 7. Specifically, in the plasma treatment tool 7, two flow paths, i.e. , a supply line 31 through which the saline is supplied to the plasma treatment tool 7 and a suction line 32 through which the saline including the transpired body tissue is sucked from the probe 7b of the plasma treatment tool 7 are provided.

The supply line 31 is a line through which the saline is supplied to the plasma treatment tool 7, a supply pump (a supplying section) 33, a temperature adjustment section 34, and a supply amount sensor 35 are continuously provided on the flow path from the upstream side, and they are coupled by a supply tube 39. It is to be noted that the supply line 31 and the suction line 32 may be separately provided from the plasma treatment tool 7.

The supply pump 33 takes in the fresh saline from the saline bag 4, and supplies a supply amount designated by the control section 2 to the probe 7b of the plasma treatment tool 7. Although this supply pump 33 is not restricted in particular, a so-called roller pump which is configured to be readily managed and maintained from a hygiene standpoint and which transports a liquid present inside while squashing an elastic tube by rotation of the roller is preferably used.

A member which supplies the saline is not restricted to the saline bag 4, and it may be mounted in a container which can measure the saline (e.g. , a graduated container) at the time of manufacture. On the other hand, a suction amount sensor (a flow volume sensor) is arranged on the upstream side of the suction pump (a sucking section) 36, and the saline sucked from the probe 7b through the suction tube 21 is allowed to pass through by the suction pump (the sucking section) 36, and a suction amount is measured. Besides the suction amount sensor, the saline sucked by the suction pump (the sucking section) 36 may be accommodated in a measurement enabling container. The control section 2 detects a water surface height of the saline in the container by using movement of a floated float or an optical sensor, and it is calculated into a volume of the collected saline. Alternatively, it is calculated into a volume of the same from a weight of the container. A supply amount to be supplied and a suction amount to be sucked can be detected from changes in volume of the saline.

The temperature adjustment section 34 cools or heats the saline supplied from the supply pump 33, and adjusts it to a temperature in a predetermined range suitable for an operation. Further, the saline also has a function of cooling the probe tip portion including the electrodes 15 and 16.

The supply amount sensor (a detecting section) 35 is one of publicly known flow volume sensors, and it detects a supply amount of the saline supplied to the probe 7b and transmits a supply value signal of the detected amount to the control section 2. It is to be noted that this embodiment is a structural example where the temperature adjustment section 34 and the supply amount sensor 35 are arranged on the flow path in the mentioned order from the upstream side, but it is also possible to adopt a structure putting a value on temperatures of the saline in which the supply amount sensor 35 is arranged on the upstream side to counterchange its position with that of the temperature adjustment section 34.

On the suction line 32, a suction amount sensor 38, a temperature sensor 37, and a suction pump 36 are arranged in the mentioned order from the probe 7b on the upstream side, and the saline sucked from the probe 7b by the suction pump (the sucking section) 36 is accommodated into the drain tank 6.

The suction pump 36 is coupled with the plasma treatment tool 7 by a suction tube 40, and sucks the irrigation layer of the saline including the transpired body tissue used for irrigation on the tip of the probe 7b. The suction amount sensor 38 detects a suction amount of the saline drained by suction of the suction pump 36, and transmits it as a suction value signal to the control section 2.

That is, the sensors (the detecting sections) 35 and 38 detect a supply amount of the saline (the conducive liquid solution) supplied from the supply ports (the supply section) 18 and a suction amount of the saline sucked into the suction port (the sucking section) 19, and output signals to the control section 2. It is to be noted that the temperature sensor 37 detects a temperature of the saline after detection of the suction value signal, and transmits it as a temperature signal to the control section 2.

The supply and the suction of the saline in the plasma treatment section 1 will now be described.

First, an amount of the saline required for a plasma treatment is a water amount provided by multiplying a distance L between the surface of the body tissue 41 shown in FIG. 3 and the tip surface 7c of the probe 7b by an area A of the tip surface 7c including both the electrodes 15 and 16, and the water amount which forms the irrigation layer at the tip of the probe 7b is determined as M1. That is, the water amount M1 is a water amount defined as a distance with which the active electrodes 15 are close to the body tissue 41 without touching and the plasma generating region is close to the body tissue 41 to effect transpiration at the time of generating the plasma.

That is, the supply amount or the suction amount is determined by a product of the distance L from the tip surface (the tip portion) 7c of the probe 7b to a surface layer of the body tissue on which the generated plasma acts and the area A of the irrigation layer consisting of the saline (the conductive liquid solution) on the basis of the arrangement of the active electrodes 15 and the return electrodes 16. As regards the distance L, a measuring instrument based on light irradiation may be used. Furthermore, an arch-like card protruding beyond the active electrodes 15 and the return electrodes 16 may be provided at a position outside these electrodes. This card is formed outside the plasma region, and a height thereof is set to a distance required for production of plasma discharge. Alternatively, an impedance value acquired at the time of excellent plasma generation after start of a treatment is assumed to be acquired when the distance is appropriate rather than actual measurement of the distance L. This impedance value is read and set as a reference value of an appropriate distance, and the distance between a treatment target and the electrodes of the probe 7b may be changed so that the impedance value detected during a subsequent treatment can approximate the reference value of the distance. In implementation, a range in which the set reference value falls is set in advance, and an indication using a green lamp may be performed if a detected value falls within this range, or indication using a red lamp may be performed if the detected value is outside this range, thereby informing an operator or the like.

When the active electrodes 15 move close to the body tissue 41 to a maximum extent and the body tissue can be transpired using the plasma, a minimal required water amount Mmin is provided. This minimal required water amount Min is determined as a target amount M of a water amount adjusted on the basis of a supply amount and a suction amount. This target amount M is set by the control section 2 on the basis of the area (or an immersion range) A and the distance (a minimum distance on which the plasma acts) L. That is, the control section 2 also functions as a setting section. That is, the setting section of the control section 2 can set, to a predetermined ratio, a supply amount of the saline supplied from the supply ports (the supplying section) 18 through which the saline (the conductive liquid solution) is subjected to irrigation so that the respective electrodes 15 and 16 are immersed and a suction amount of the saline sucked into the suction port (the sucking section) 19 through which the saline which has been used for irrigation and passed through the respective electrodes 15 and 16.

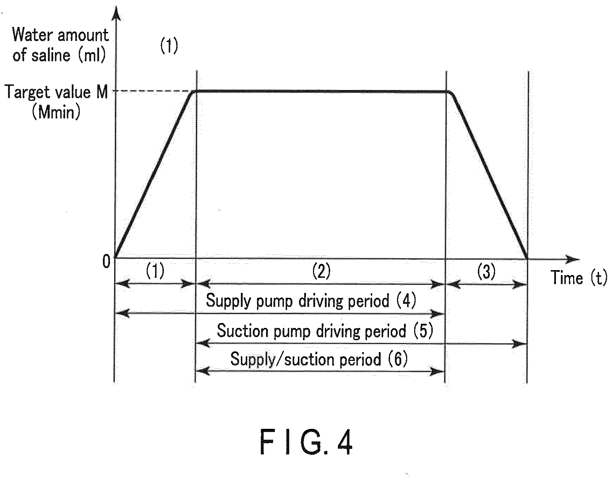

FIG.4 is a view showing a relationship between a supply amount and a suction amount of the saline as the target amount M of an amount of the saline.

As shown in FIG. 4, as changes in supply amount of the saline according to this embodiment, the saline is supplied (fed) and increased until the target amount M is reached, then suction is gradually started at the target amount M, and the target amount M is maintained. After maintaining this target amount M, a plasma treatment is started. After end of the plasma treatment, the saline in the irrigation layer formed on the probe 7b is completely sucked.

Specifically, periods (1) and (2) shown in FIG. 4 are a supply pump driving period (4) during which the supply pump 33 which supplies the saline is driven, and periods (2) and (3) are a suction pump driving period (5) during which the suction pump 36 which sucks the saline is driven. Among others, during the period (1), timing to start the treatment is hastened as an initial rise from 0 to the target amount M is quick. Further, the shared period (2) is a supply/suction period (6) during which both the pumps are driven so that a water amount of the irrigation layer of the saline formed on the probe tip portion is maintained at the target amount. During this supply/suction period (6), plasma is generated at the active electrodes 15 to perform the plasma treatment. After stopping the plasma, the supply pump 33 is stopped, driving of the suction pump 36 is continued, and the saline remaining on the tip portion of the probe 7b is all sucked, thereby terminating the plasma treatment.

It is to be noted that the control section 2 controls the supply pump 33 and the suction pump 36 so that the supply amount and the suction amount become substantially equal to each other by irrigating the portion between the first and second electrodes 15 and 16 with the saline (the conductive liquid solution) when the plasma is generated by the high-frequency energy in a state where the saline is interposed near the electrodes 15 and 16. That is, as will be described alter, in the control section 2, a coefficient (a ratio) of the first information and the second information is 1: 1. This coefficient (the ratio) is set so that the saline which is the conductive liquid solution has the minimal water amount required for generation of the plasma.

Furthermore, the setting section which sets a supply amount of the saline supplied from the supply pump (the supplying section) 33 which performs irrigation using the saline (the conductive liquid solution) from the second electrodes 16 toward the first electrodes 15 so that both the first and second electrodes 15 and 16 are immersed and a suction amount of the saline sucked into the suction pump (the sucking section) 36 which sucks and discharges the saline which has been subjected to irrigation and has passed through the first and second electrodes 15 and 16 to a predetermined ratio, the detecting sections 35 and 38 which detect the supply amount of the saline supplied from the supply pump 33 and the suction amount of the saline sucked into the suction pump 36, and the control section 2 which controls the supply amount and the suction amount to the ratio set by the setting section on the basis of the supply amount (a water feed amount) and the suction amount detected by the detecting sections 35 and 38 constitute a control system. In other words, the control system includes the control section 2 which compares the supply amount detected by the supply amount sensor 35 with the suction amount detected by the suction amount sensor 38 to perform feedback control and carries out control so that the supply amount and the suction amount can reach the preset target amount M.

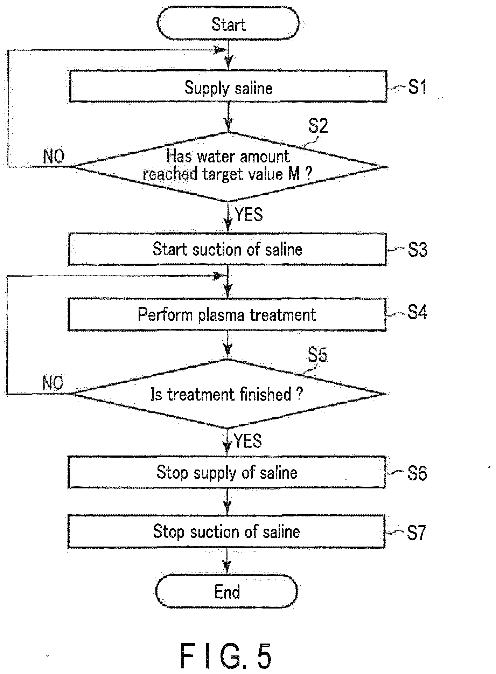

A procedure of the plasma treatment in the plasma treatment tool 7 according to this embodiment will now be described with reference to a flowchart shown in FIG. 5.

Before starting the treatment, the tip surface 7c of the plasma treatment tool 7 is arranged at a position close to a treatment target (a narrow gap), the water feed switch in the switches 8 is operated to drive the supply pump 33 , and the saline is stagnated at the tip portion of the probe 7b (a step S1). Then, whether a stagnated water amount is maintained at the target amount M is determined (a step S2) . Supply of the saline is continued when the water amount does not reach the target amount M (NO), or the suction pump 36 is driven, irrigation is performed while keeping the balance of the supply amount and the suction amount so that the saline does not overflow from the tip surface 7c of the probe 7b as will be described later, and the water amount which is the target amount M is maintained when the water amount has reached the target amount M (YES) (a step S3).

Then, the operator operates the footswitch to apply high-frequency power to the active electrodes 15 at the tip of the probe 7b from the power supply section 3, generates the plasma, and executes a treatment of transpiring a desired body tissue (a step S4). The control section 2 determines whether the operator has terminated the treatment (a step S5), namely, determines whether the footswitch has been turned off and, in case of OFF (YES), the control section 2 stops application of the high-frequency power to terminal the plasma treatment, annihilates the plasma, stops the supply pump 33 (a step S6), drives the suction pump 36 only, estimates timing (a predetermined time) of terminating the suction of the saline used for irrigation on the tip of the probe 7b, and then stops the suction pump 36 (a step S7).

Such an operation is repeatedly carried out every time the plasma treatment is performed. It is to be noted that the irrigation of the saline may be continued until the treatment is completed and the supply may be controlled to maintain the target amount M or, usually, the irrigation may be stopped and an irrigation amount of the saline may be set to the target amount M every time the plasma is generated. Further, the footswitch of the operation may substitute for the water feed switch, and the high-frequency power may be applied when the water amount which is the target amount M is reached.

Flow volume control of the supply amount and the suction amount of the saline in the first embodiment will now be described. To facilitate the following description, it is assumed that a flow volume sensor having the same specification as those of the supply amount sensor and the suction amount sensor can provide a detection signal having the same value if a flow volume remains the same. As a matter of course, if a detection signal value to a passing flow volume is defined, a water amount to be supplied can be easily compared with a water amount to be sucked by correcting the signal value.

In this embodiment, the supply pump 33 is first driven to supply the saline so that a water amount of the irrigation layer formed on the tip surface 7c of the probe 7b becomes the target amount M. When the supply amount (an accumulated amount) reaches the target amount M, driving of the suction pump 36 is started, and the same suction amount as the supply amount is sucked to adjust the flow volume. Specifically, an output from the supply amount sensor is determined as a flow volume signal A (a supplied water amount), an output from the suction amount sensor is determined as a flow volume signal B (a sucked water amount: a feedback signal), a difference signal of the flow volume signal B to the flow volume signal A is taken, and the feedback control is repeatedly performed so that the difference signal constantly approximates zero. Specifically, when the driving of the suction pump 36 is changed to adjust the output of the flow volume signal B so that the fixed flow volume signal A can be acquired, the same driving state of the supply pump 33 can be maintained.

Further, when a driving state (the supply amount) of the supply pump has changed, the suction amount of the suction pump is equivalently changed like synchronization. In case of such control, if the fixed target amount M is maintained as long as the plasma state can be maintained, raising the supply amount of the supply pump results in proportionately increasing the suction amount of the suction pump. Consequently, a flow rate of the irrigation using the saline increases. That is, a period of stagnation on the probe tip is shortened, and recovery of the saline containing impurities (the transpired body tissue) after the treatment can be hastened.

Thus, according to this embodiment, the flow volumes to be supplied and sucked can be independently adjusted so that the supply amount of the saline supplied from the supply pump 33 to the plasma treatment tool 7 can be always equal to the suction amount recovered from the saline from the plasma treatment tool 7 after the treatment. Therefore, a water amount used for irrigation on the tip of the probe 7b can be arbitrarily set by supplying the desired supply amount and performing the water amount adjustment of sucking the supplied water amount, thereby adjusting to a minimal required water amount Mmin.

Thus, according to the system 1 of this embodiment, for example, a transpiring treatment of a tonsil, since substantially all of the supplied saline can be sucked, it is possible to avoid pulmonary aspiration that the saline enters a bronchial tube or the like without being sucked as much as possible.

It is to be noted that the example where the area A is adopted as mentioned above has been described in this embodiment. The area A can be appropriately set. For example, an area of the tip surface 7c of the probe 7b may be directly determined as the area A.

### [Modification of First Embodiment]

A modification of the first embodiment will now be described.

This modification is an example of detecting clogging of the suction port by using the configuration of the plasma treatment system 1 shown in FIG. 1 to FIG. 3 according to the first embodiment.

The suction port 19 provided in the tip surface 7c of the probe 7b in the plasma treatment tool 7 sucks the saline used for irrigation of the tip surface 7c by using the suction pump 36 and discharges it to the drain tank 6. As described above, the saline sucked from the suction port 19 contains a body tissue transpired by plasma. Since the body tissue is usually destroyed and transpired by the plasma, a micro-sized body tissue floats, and a tiny piece of body tissue may be contained depending on how the transpiration is carried out. When a size of this tiny piece of body tissue is larger than a diameter of the suction port 19 or many tiny pieces of body tissue are simultaneously sucked, or when the body tissue is caught depending on an inner diameter of an inner tube or how the tube bends, it can be considered that clogging occurs in the suction line 32.

At this time, when an amount of the flowing saline is lowered, a signal value of the flow volume signal B output by the suction amount sensor is also decreased. In the control section 2, in a state where a threshold value (a judgment value) is provided to the flow volume signal B in advance, a normal water amount is supplied, and the suction pump normally drives, when the signal value of the flow volume signal B falls below the threshold value, the amount of the passing saline is reduced, and hence clogging is determined to have occurred in the suction line 32.

According to this modification, since the threshold value is set to the signal value of the flow volume signal B output from the suction amount sensor, when the flow volume signal B falling below this threshold value is generated, application of the high-frequency power can be stopped, then driving of the supply pump 33 can be stopped, supply of the saline can be stopped, and the plasma treatment can be safely stopped. Furthermore, when a non-illustrated warning function is mounted, an operator can be informed of occurrence of abnormality if the supply pump 33 is stopped in a state where the switches 8 are ON. When this notification is delivered to the operator, a reminder can be promoted about an abnormal state of the treatment tool.

### [Second Embodiment]

A second embodiment will now be described hereinafter.

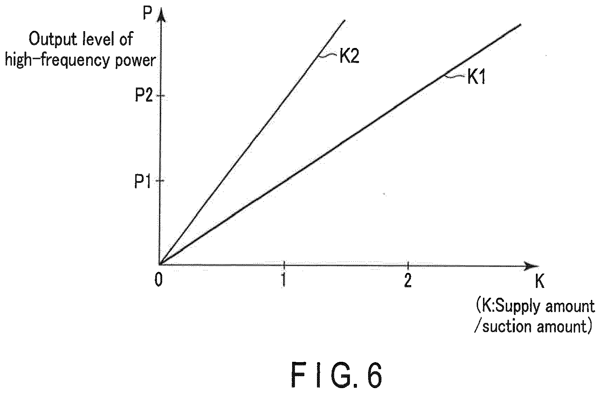

FIG. 6 is a view showing a relationship between two characteristic lines (K1 and K2), each of which is provided by a coefficient K as a ratio of water amounts of a supply amount and a supply amount of saline in each of first and second plasma treatments, and an output level of high-frequency power. The coefficient K is previously recorded in the setting section provided in a control section 2, and characteristics formed of a linear primary expression is shown as an example here. However, curved characteristics formed of a quadratic or characteristics which vary in a step-like manner may be provided. A value of this coefficient K can be probatively or empirically obtained on the basis of a specification of a probe (a design specification such as a shape of a tip, a shape of an electrode, diameters or arrangement positions of a supply port and a suction port, or a value of high-frequency power to be applied) in a design phase of a treatment tool.

A necessary amount of the saline to be used differs depending on an output level of the high-frequency power applied in the plasma treatment. Furthermore, the supply amount is not necessarily equal to the suction amount. During immersion of active electrodes 15 and return electrodes in an irrigation layer of the saline which irrigates a tip portion of the probe in a preferred state, even if evaporation and consumption are performed by generated plasma and all of the saline is sucked, the suction amount (second information in the control section 2) is smaller than the supply amount (first information in the control section 2) in some cases. In a first plasma treatment tool having the characteristics K1 in the example shown in FIG. 6, the coefficient K1=1 is achieved, i.e., the supply amount is equal to the suction amount when the output level is P1. That is, in the control section 2, a coefficient (a ratio) of the first information and the second information is 1:1. This coefficient (the ratio) is set so that the saline which is a conductive liquid solution has a minimal water amount required for generation of the plasma.

When this ratio is used, as a first control system, to realize a plasma treatment selected by an operator or the like, information of the ratio stored in the control section 2 can be read out from the output level of the high-frequency power to be applied, and the required supply amount and suction amount of the saline can be set in the supplying/sucking section 5. Additionally, as a second control system, information of a ratio detected from the supply amount and the suction amount of the saline actually used for irrigation of the probe 7b can be assigned to the information of the ratio stored in the control section 2, and a value of the high-frequency power applied to the active electrodes 15 of the probe 7b can be set.

Further, this system 1 can be used for control in a situation where the output level of the high-frequency increases or decreases on the way. That is, the ratio can be changed on the basis of an energy amount of the high-frequency energy. For example, when the output level increases and reaches P2 which is double P1, it is possible to make reference to the coefficient K and change to the set supply amount and suction amount. For example, the coefficient K1=2, namely, the supply amount is double the suction amount. In this manner, with given characteristics K1, the coefficient K according to a straight line having a preset inclination or a curved line can be obtained on the basis of a change in output level P, and the saline is supplied and sucked with a ratio of the supply amount and the suction amount based on this coefficient K. The characteristics K2 are different from the characteristics K1 in an output level of the high-frequency power to be applied due to differences in shape of electrodes or in a supply amount of the saline, and equivalent control is basically carried out.

Thus, according to this embodiment, the output level of the high-frequency power applied to the probe of the plasma treatment tool is controlled in correspondence with the supply amount and the suction amount of the saline on the tip portion of the probe. Furthermore, when an operator just selects an output level of the high-frequency power corresponding to a plasma treatment by using switches or the like, a ratio of the supply amount and the suction amount of the saline which is a conductive liquid solution is set, and the tip surface 7c of the probe 7b is irrigated, and an irrigation layer made up of a minimal required water amount is formed between the tip surface 7c and a treatment target, and the plasma treatment is performed.

### [Third Embodiment]

A third embodiment will now be described.

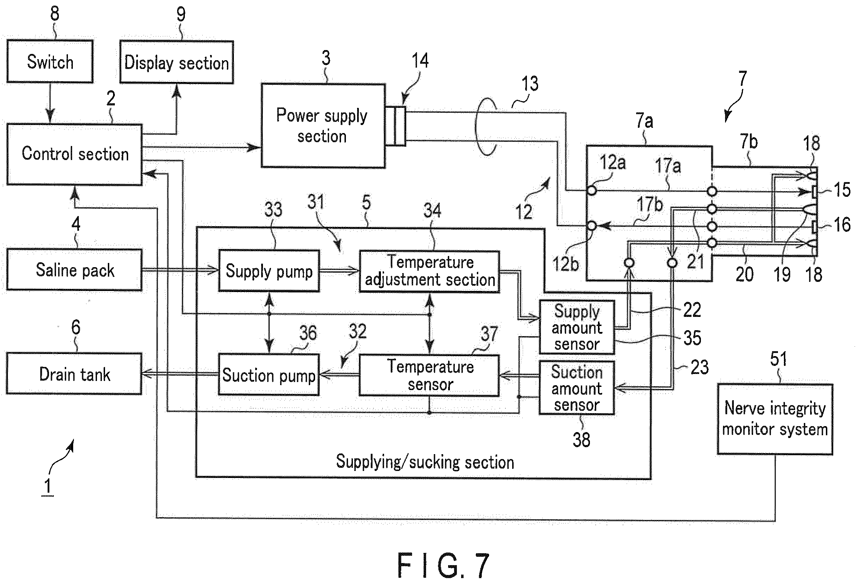

FIG. 7 shows a structural example of this embodiment provided by adding a nerve integrity monitor system 51 to the configuration of the plasma treatment tool system depicted in FIG. 1.

The nerve integrity monitor system 51 is a publicly known system. In general, monitor electrodes (not shown) are attached to an innervated muscle near a body tissue which is a treatment target during a surgical operation, stimulus signals of a constant-current output from a stimulation apparatus (not shown) are applied as needed, and whether a nerve is affected by the surgical operation or a treatment is determined from sound or an image (a waveform) based on a signal acquired from the monitor electrodes. Based on this determination, a probe 7b of a plasma treatment tool 7 is operated while confirming that a normal nerve is not damaged, and an irrigation treatment of the treatment target is performed.

If presence of the nerve was confirmed in this determination, a warning signal is supplied to a control section 2 from the nerve integrity monitor system 51, and warning sound is given or warning display is performed. Upon receiving this warning signal, the control section 2 stops application of high-frequency power to the probe 7b from a power supply section 3, stops a supply pump 33, and also stops supply of saline. Moreover, after a predetermined time, the control section 2 stops a suction pump 36, and interrupts the treatment.

As described above, according to this embodiment, when the nerve which is affected by the plasma treatment is confirmed by monitoring the nerves near the treatment target as needed during the plasma treatment, heat invasion to the nerve can be avoided by giving a warning and interrupting the plasma treatment without the operator's stop operation, thereby reducing pain after the surgical operation.

### [Fourth Embodiment]

A fourth embodiment will now be described.

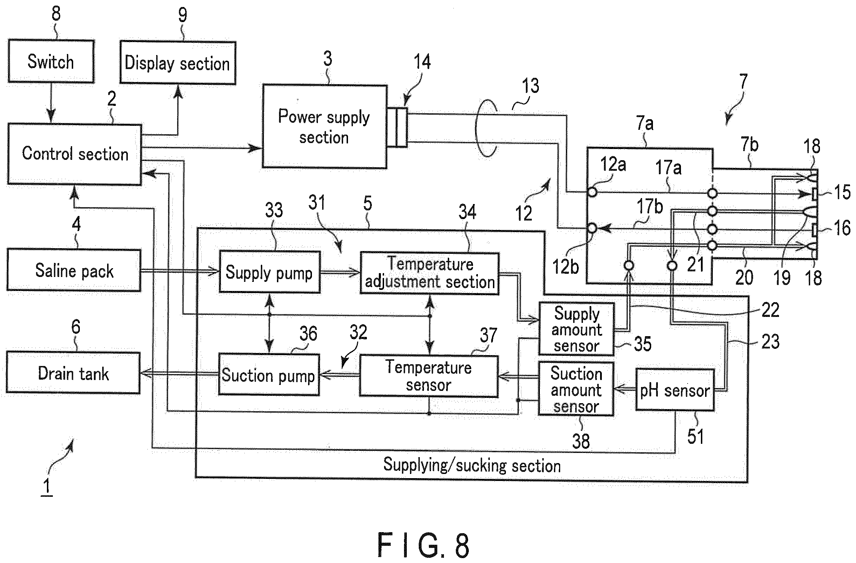

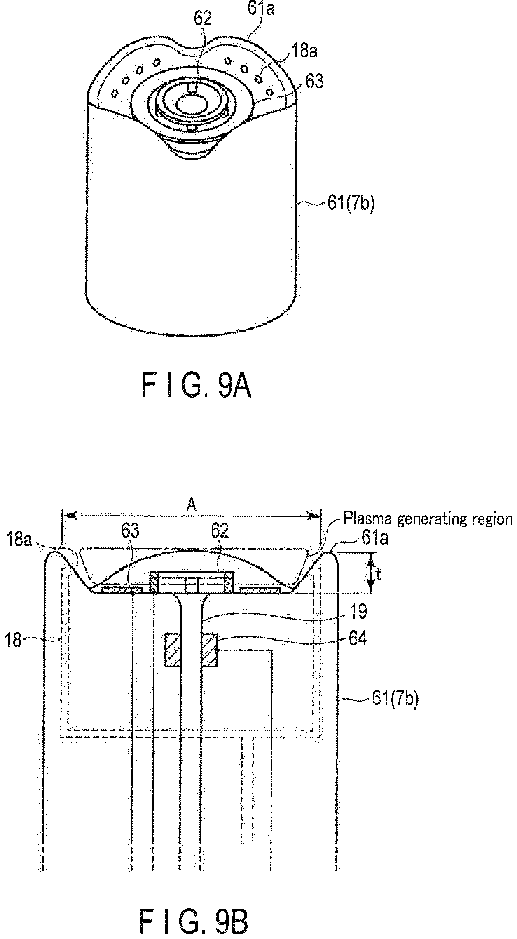

FIG. 8 shows a structural example of this embodiment provided by adding a pH (a hydrogen ion concentration index) sensor having a publicly known detecting configuration to the configuration of the plasma treatment tool shown in FIG. 1. FIG. 9A shows an appearance configuration of a tip portion of a probe 61 (7b) of a plasma treatment tool according to a fourth embodiment, and FIG. 9B shows a cross-sectional view of a tip side of the probe 61.

In general, after a plasma treatment, since a body tissue (a mucous membrane, a viscous liquid, or the like) transpired into saline is mixed, a pH level varies to the pure saline before the treatment. According to this embodiment, this change in pH is detected, a treatment state is grasped, and an increase or a decrease in a supply amount and a suction amount of the saline, control over high-frequency power to a plasma generation state, and others are carried out.

This probe 61 has a cylindrical shape, its tip surface is recessed into a concave shape, and four continuous corrugated protruding portions 61a are formed to extend on a circumference in this example. As shown in FIG. 9B, these protruding portions 61a have a height (a distance) t from a tip surface to a protruding top portion set to be equal to the distance L which is defined in the plasma generating region and described in the first embodiment. A suction port 19 is opened at the center of the tip surface. Supply ports 18 are continuously opened in inner peripheral side surfaces of the protruding portions 61a. A ring-shaped active electrode 62 which is supported by support portions to be apart from the tip surface is provided around the suction port 19. Further, a ring-shaped return electrode 63 is provided outside the support portions on the tip surface. The saline is subjected to irrigation from supply ports 18 toward the suction port 19 at the center of the tip surface, and the same irrigation layer as that in the first embodiment is formed.

It is to be noted that the protruding portions 61a enable taking a distance between the active electrode 62 and the body tissue. Thus, high-frequency energy can be prevented from entering the body tissue in a state where the active electrode 62 is in direct contact with the body tissue. Furthermore, since the protruding portions 61a have recessed portions, it is possible to prevent the probe tip surface, the protruding portions 61a, and the body tissue from forming a closed space. Thus, suction through the suction port 19 can prevent the body tissue from coming into contact with the active electrode 62.

The pH sensor may be arranged near the active electrode 62, but a situation where the upper side of the pH sensor is not irrigated with the saline having the body tissue mixed therein can be also considered depending on a direction along which the probe 61 moves. In this embodiment, as shown in FIG. 9B, the pH sensor 64 is provided to surround a periphery of a suction pipe at a position internally introduced from the suction port 19. Usually, pH of the saline has acid characteristics of approximately 5.5 to 6. On the basis of such characteristics, when the body tissue (e.g., approximately pH 7.4 in case of a body fluid, blood, or the like) as an impurity is mixed in the saline by a plasma treatment, a value of pH increases, namely, the characteristics verge on alkaline properties. Thus, to purify the saline (restore a reference value of pH), processing is carried out to increase a supply amount or a suction amount. Further, to finely divide the chipped-off body tissue and facilitate suction, a level of high-frequency output may be increased to raise plasma density.

In this embodiment, there is shown an example where a portion coupling the suction port 19 with the inner tube is made of glass and it is integrally formed with the pH sensor, whose periphery is made of glass, along the path. This arrangement enables the saline having the body tissue mixed therein to assuredly pass through the pH sensor at the time of suction, and hence detection can be securely carried out. A control section 2 includes a table which stores patterns of an increase/decrease in output level of the high-frequency power or an increase/decrease in saline in correspondence with a pH level, and a preset pattern is selected on the basis of the pH level detected by the pH sensor, thereby performing each control.

As described above, according to this embodiment, since the pH sensor is disposed at a position internally introduced from the suction port 19, the saline containing the treated body tissue can be passed, and a pH level can be measured. A current state of a plasma treatment can be grasped from this measurement value, and it is possible to perform, e.g., control of the high-frequency power to an increase/decrease in a supply amount and a suction amount of the saline or a plasma generation state. Furthermore, increase/decrease values of the supply amount or the suction amount of the saline on the treatment tool side or a set value to switch output levels of the high-frequency power may be determined in, e.g., the table in the control section 2.

Moreover, in the probe, since the protruding portions 61a having a height which enables formation of an irrigation layer of a predetermined water amount are formed at the circumference of the tip surface, an operator can assure a space for a treatment (the plasma generating region) by just pressing the probe tip. Additionally, since the plasma generating region can be restricted by the protruding portions, even if a nerve and others are present near a treatment target position, the treatment can be performed even to the vicinity of the nerve.

The respective embodiments and modifications described above include the following gist of the invention.
1. A plasma treatment system which supplies high-frequency energy to a body tissue, the system including:
   a treatment tool having a pair of bipolar electrodes which supply the high-frequency energy to the body tissue;
   a supplying section which is provided in the treatment tool, and supplies a conductive liquid solution to generate plasma toward a surface layer of the body tissue to be treated from the treatment tool;
   a sucking section which is provided in the treatment tool, and sucks the conducive liquid solution;
   a setting section which sets a predetermined ratio as a proportion of predetermined first information of the conductive liquid solution supplied from the supplying section and predetermined second information of the conductive liquid solution sucked into the sucking section;
   a detecting section which detects a supply amount of the conductive liquid solution supplied from the supplying section and a suction amount of the conductive liquid solution sucked into the sucking section; and
   a control section which controls supply of the high-frequency energy to the body tissue on the basis of the predetermined ratio from a ratio of the supply amount and the suction amount detected by the detecting section.
2. The plasma treatment system according to Section (1), characterized in that the detecting section is linked with a calculating section which is electrically connected thereto and calculates a volume from a weight to the supply amount or the suction amount or a height of a liquid level in a measuring instrument for measurement of the conductive liquid solution.
3. The plasma treatment system according to Section (1), characterized in that the supply amount or the suction amount is an amount determined by a distance between a surface layer area of the body tissue as a treatment target and the bipolar electrodes.
4. The plasma treatment system according to Section (1), characterized in that the control section is electrically connected to a first sensor which detects presence/absence of a nerve tissue under the surface layer of the body tissue, and
   the control section controls to stop at least one of the supplying section, the sucking section, and supply of the high-frequency energy when the nerve tissue is present.
5. The plasma treatment system according to Section (1), characterized in that the control section is electrically connected to a second sensor which detects information indicating that an opening provided in a tip portion of the sucking section or a channel communicating with a proximal end portion of the treatment section from the opening is clogged with the liquid solution or the treatment target, and
   the control section controls to stop at least one of the supplying section, the sucking section, and supply of the high-frequency energy on the basis of the second sensor information indicating that the opening is clogged.
6. The plasma treatment system according to Section (1), characterized in that the control section controls the supplying section and the sucking section so that a ratio of the supply amount and the suction amount becomes 1 to 1.
7. A plasma treatment system which applies high-frequency energy to a body tissue, including:
   a treatment tool which is extended from a proximal end portion toward a tip portion, and gives a treatment to the body tissue by providing a pair of bipolar electrodes, which supply the high-frequency energy to the body tissue, on the tip portion;
   a supplying section which is provided in the treatment tool, and supplies a conductive liquid solution from the tip portion toward a surface layer of the body tissue to be treated;
   a sucking section which sucks the supplied conductive liquid solution from the tip portion toward to the proximal end portion of the treatment tool;
   a detecting section which detects pH of the conductive liquid solution sucked by the sucking section;
   a setting section which sets a previously set predetermined pH of the conducive liquid solution when it is sucked by the sucking section;
   a temperature adjusting section which is provided in the treatment tool, and adjusts a temperature of the conducive liquid solution to increase or decrease a temperature of the conductive liquid solution supplied from the supplying section;
   a first control section which compares the pH of the conductive liquid solution obtained by the detecting section with a threshold value of the predetermined pH of the conductive liquid solution, and controls the temperature adjusting section so that a temperature of the conductive liquid solution corresponding to the predetermined pH is reached; and
   a second control section which controls a supply amount of the high-frequency energy to the body tissue from the temperature of the conductive liquid solution controlled by the first control section.
8. The plasma treatment system according to Section (7), characterized in that the second control section controls the supply amount of the conductive liquid solution from the supplying section or the suction amount of the conductive liquid solution into the sucking section.
9. The plasma treatment system according to Section (7), characterized in that the detecting section detects pH of the conductive liquid solution by using a first sensor provided in a channel of the sucking section communicating with the proximal end portion from the tip portion.
10. The plasma treatment system according to Section (8), characterized in that the second control section controls to stop application of the supply amount and the suction amount and application of the high-frequency energy supplied to the body tissue when a temperature of the conductive liquid solution sucked into the sucking section by suction exceeds the predetermined temperature in the heating section.
11. A control system which is used with a plasma treatment tool including:
   a first electrode; and
   a second electrode which forms a pair to generate plasma by using supplied high-frequency energy, and is arranged around the first electrode, the control system including:
      a setting section which sets, to a predetermined ratio, a supply amount of the conductive liquid solution which is supplied from a supplying section to immerse both the first and second electrodes from the second electrode toward the first electrode and a suction amount of the conductive liquid solution sucked by irrigating the first and second electrodes;
      a detecting section which detects a supply amount of the conductive liquid solution supplied from the supplying section and a suction amount of the conductive liquid solution sucked into the sucking section; and
      a control section which controls the supply amount and the suction amount to the ratio set by the setting section on the basis of the supply amount and the suction amount detected by the detecting section.

According to the present invention, it is possible to provide the plasma treatment system including the supply/suction unit which supplies and sucks a minimal required conductive liquid solution to and from the treatment tool which generates plasma in the conductive liquid solution to give a treatment to a body tissue.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as def ined by the appended claims and their equivalents.

## Claims

1. A plasma treatment system **characterized by** comprising:
a treatment section which comprises a first electrode and a second electrode which forms a pair with the first electrode, and generates plasma in a conductive liquid solution, in which the first electrode and the second electrode are immersed, by using high-frequency energy supplied between the first electrode and the second electrode to give a treatment to a body tissue;
a setting section which sets a predetermined ratio of a supply amount from a supplying section which supplies the conductive liquid solution for immersion between the first electrode, the second electrode, and a treatment target surface layer of the body tissue, and a suction amount from a sucking section which sucks the conductive liquid solution;
a detecting section which detects a supply amount of the conductive liquid solution supplied from the supplying section, and a suction amount of the conductive liquid solution sucked into the sucking section; and
a control section which controls the supply amount and the suction amount to the ratio set by the setting section on the basis of the supply amount and the suction amount detected by the detecting section.

2. The system according to claim 2,
**characterized in that** the suction amount supplied from the supplying section and the suction amount sucked into the sucking section are amounts determined by a product of a distance from a tip portion of a probe of the treatment tool, on which the first electrode and the second electrode are provided, to a surface layer of the body tissue on which the generated plasma acts, and an area of an irrigation layer consisting of the conductive liquid solution by arrangement of the first electrode and the second electrode.

3. The system according to claim 1,
**characterized in that** the ratio is changeable on the basis of an energy amount of the high-frequency energy.

4. The system according to claim 1,
**characterized in that** the control section makes the supply amount and the suction amount equal to each other while a portion between the first electrode and the second electrode is irrigated with the conducive liquid solution when the plasma is generated by using the high-frequency energy.

5. A plasma treatment system which is a control system used together with a supplying/sucking section **characterized by** including:
a plasma treatment tool having a probe which comprises at a tip portion thereof a first electrode and a second electrode to generate plasma from high-frequency energy;
a high-frequency power supply which supplies the high-frequency energy to the first electrode and the second electrode;
a supplying section which performs irrigation with a predetermined supply amount of a conductive liquid solution provided by a supply amount sensor so that both the first electrode and the second electrode are immersed in the conductive liquid solution; and
a sucking section which sucks and discharges a predetermined suction amount of the conductive liquid solution that has been used to irrigate and has passed the first electrode and the second electrode from the suction port and that is provided by a suction amount sensor,
the system comprising a control section which compares a supply amount detected by the supply amount sensor with a suction amount detected by the suction amount sensor, performs feedback control, and controls the supply amount and the suction amount to reach a predetermined target amount within an immersion range to immerse both the first electrode and the second electrode.

6. The system according to claim 5,
**characterized in that** the supply amount or the suction amount is an amount determined by a product of a distance from a tip portion of the probe to a surface layer of a body tissue as a treatment target on which the generated plasma acts, and an area of an irrigation layer consisting of the conductive liquid solution by arrangement of the first electrode and the second electrode.

7. The system according to claim 5, **characterized by** further comprising a pH sensor which is arranged at a position internally introduced through the suction port of the probe and detects a hydrogen ion concentration index pH of the conductive liquid solution after a treatment sucked from the suction port.
